# EUROPÄISCHE PATENTSCHRIFT

(11) **EP 0 671 381 B1**
(45) Veröffentlichungstag und Bekanntmachung des Hinweises auf die Patenterteilung: **14.01.1998**
(21) Anmeldenummer: 95102761.4
(22) Anmeldetag: 27.02.1995
(51) Int. Cl.: C07C 205/44, C07C 201/12

(54) **Verfahren zur Herstellung von 2-Nitrobenzaldehyden**
Process for the preparation of 2-nitrobenzaldehydes
Procédé pour la préparation de 2-nitrobenzaldéhydes

(30) Priorität: 10.03.1994 DE 4408007
(43) Veröffentlichungstag der Anmeldung: 13.09.1995
(73) Patentinhaber: BAYER AG, 51368 Leverkusen (DE)
(72) Erfinder: Schnatterer, Albert, Dr., D-51373 Leverkusen (DE); Fiege, Helmut, Dr., D-51373 Leverkusen (DE)

(56) Entgegenhaltungen:
- DATABASE WPI Section Ch, Week 9414 Derwent Publications Ltd., London, GB; Class E14, AN 94-116609 & SE-A-9 202 203 ( CHEM DYNAMICS DEV AB) , 21.Januar 1994

## Beschreibung

Die vorliegende Erfindung betrifft ein Verfahren zur Herstellung von 2-Nitrobenzaldehyden durch Oxidation von 2-Nitrotoluolen mit Sauerstoff.

2-Nitrobenzaldehyde sind bedeutende Zwischenprodukte für Pharmazeutika. Der 2-Nitrobenzaldehyd ist beispielsweise Ausgangsprodukt für den in der Koronartherapie eingesetzten Wirkstoff Nifedipin.

Zur Herstellung von 2-Nitrobenzaldehyden ist bereits eine größere Anzahl von Verfahren bekannt. Die vom wirtschaftlichen Standpunkt aus interessantesten Verfahren gehen von 2-Nitrotoluol aus. 2-Nitrotoluol ist großtechnisch verfügbar und stellt eine besonders preisgünstige Basis dar. Als desaktiviertes Derivat des Toluols läßt 2-Nitrotoluol allerdings nur eine geringe Reaktivität gegenüber einem oxidativen Angriff an der Methylgruppe erwarten. In Übereinstimmung mit dieser Aussage ist die direkte Oxidation von 2-Nitrotoluol zu 2-Nitrobenzaldehyd bisher nur mit Metallen in hohen Oxidationsstufen als Oxidationsmittel beschrieben, beispielsweise mit Ce(IV) in Perchlorsäure als Lösungsmittel (vgl. z.B. EP-A 205 173) oder mit elektrochemisch erzeugtem Co(III) in Schwefelsäure als Lösungsmittel (vgl. J. Appl. Elektrochem. 9 (1979), 96, 753-5). Der stöchiometrische Einsatz von Metallverbindungen ist aber von den Materialkosten her teuer, der Prozeß der elektrochemischen Regenerierung ebenso wie die Handhabung größerer Mengen dieser Stoffe aufwendig. Außerdem lassen die Lösungsmittel Schwefelsäure und Perchlorsäure erhebliche Kosten bei der Entsorgung bzw. Recyclisierung erwarten, ganz abgesehen von der Sicherheitsproblematik beim Arbeiten mit Perchlorsäure.

Um die geringe Reaktivität des 2-Nitrotoluols gegenüber Oxidationsmitteln zu umgehen, wurde in der Vergangenheit in vielen Verfahren zur Herstellung von 2-Nitrobenzaldehyd der Umweg über die Aktivierung der Methylgruppe im 2-Nitrotoluol eingeschlagen. So kann nach Umsetzung des 2-Nitrotoluols mit Brom zum 2-Nitrobenzylbromid letzterer in guten Ausbeuten zu 2-Nitrobenzaldehyd oxidiert werden, z.B. durch Reaktion mit Dimethylsulfoxid (vgl. DE-A 2 808 930) oder tertiären Aminoxiden (DE-A 2 948 058). Alternativ dazu kann die Oxidation auch von der Stufe des 2-Nitrobenzylalkohols, dem Hydrolyseprodukt des 2-Nitrobenzylbromids, aus durchgeführt werden, z.B. mit CrO₃ (vgl. Bull. Chem. Soc. Jpn. 63 (1990) 8, 2433-4) oder NH₄VO₃ (vgl. Indian J. Chem. Sect. B. 29B (1990) 3, 257-62).

Eine weitere Möglichkeit der Aktivierung des 2-Nitrotoluols beruht auf der Derivatisierung durch C-C-Verknüpfung. Über die Umsetzung von 2-Nitrotoluol mit Dimethylformamidacetal gelangt man zum 2-Amino-o-nitrostyrol, das nach EP-A 430 001 mit Sauerstoff unter Kupferkatalyse oder nach JP 60 025 957 mit Natriumhypochlorit zu 2-Nitrobenzaldehyd oxidiert werden kann. Eine weitere Variante des Verfahrens der C-C-Verknüpfung und nachfolgender Oxidation ist die Reaktion von 2-Nitrotoluol mit Oxalsäureestern zu Derivaten der Brenztraubensäure. Die anschließende Oxidation kann z.B. gemäß EP-A 92 267 mit H₂O₂ oder gemäß DE-A 2 415 061 mit Kaliumpermanganat durchgeführt werden.

Die bisher bekannten Verfahren zur Herstellung von 2-Nitrobenzaldehyd auf Basis 2-Nitrotoluol haben den Nachteil, daß entweder mit Metallverbindungen in Perchlorsäure oder Schwefelsäure oxidiert werden muß oder aber mehrstufige Prozesse in Kauf genommen werden müssen. Auf die Probleme beim Arbeiten mit Metallverbindungen in Perchlorsäure oder Schwefelsäure wurde bereits weiter oben hingewiesen. Die Nachteile mehrstufiger Verfahren liegen in den hohen Anlage- und Betriebskosten. Außerdem benötigten die genannten mehrstufigen Verfahren teure Reaktionskomponenten wie z.B. Brom oder Dimethylformamidacetal und z.T. auch noch teure Oxidationsmittel wie z.B. Dimethylsulfoxid, tertiäre Aminoxide oder Kaliumpermanganat.

Es bestand deshalb Bedarf an einem Verfahren, das die Herstellung des 2-Nitrobenzaldehyds in einem möglichst 1-stufigen Verfahren unter Verwendung eines billigen Oxidationsmittels ermöglicht.

Gegenstand der Erfindung ist ein Verfahren zur Herstellung von 2-Nitrobenzaldehyden der allgemeinen Formel 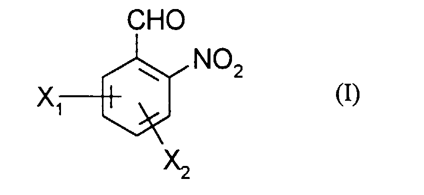 worin
- X₁ und X₂: entweder unabhängig voneinander für Wasserstoff oder Halogen stehen oder einer der Substituenten für Nitro steht und der andere Substituent dann für Wasserstoff steht,
durch Oxidation von 2-Nitrotoluolen der allgemeinen Formel 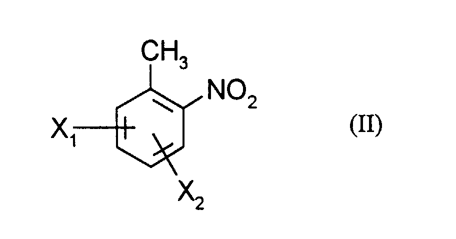 worin
- X₁ und X₂: die oben angegebene Bedeutung haben
dadurch gekennzeichnet, daß die Oxidation mit Sauerstoff oder einem Sauerstoff enthaltenden Gas in Gegenwart mindestens eines Alkoxyalkylamins als Lösungsmittel und in Gegenwart von starken Basen durchgeführt wird.

Halogen in der Definition von X₁ und X₂ steht vorzugsweise für Fluor, Chlor oder Brom. Beispielhaft seien für erfindungsgemäß einsetzbare 2-Nitrotoluole gemäß der Definition in Formel (II) genannt: 2-Nitrotoluol, 4-Fluor-2-nitrotoluol, 4-Chlor-2-nitrotoluol, 2,4-Dinitrotoluol, 2,6-Dinitrotoluol. Besonders geeignet ist das erfindungsgemäße Verfahren zur Oxidation von 2-Nitrotoluol.

Die als Lösungsmittel zur Durchführung des erfindungsgemäßen Verfahren eingesetzten Alkoxyalkylamine entsprechen vorzugsweise der Formel 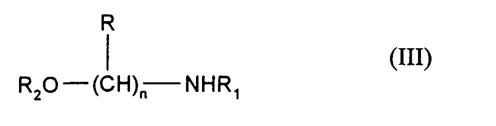 worin
- R und R₁: unabhängig voneinander für Wasserstoff oder C₁-C₄-Alkyl stehen,
- R₂: für C₁-C₄-Alkyl steht und
- n: für 1 bis 6 steht.

Beispiele für die erfindungsgemäß einzusetzenden Alkoxyalkylamine sind:

2-Methoxyethylamin, 2-Ethoxyethylamin, 3-Methoxy-1-propylamin, 3-Ethoxy-1-propylamin, 4-Methoxy-1-butylamin, 4-Methoxy-2-butylamin, N-Methyl-2-methoxyethylamin, N-Methyl-3-methoxy-1-propylamin, 2-Methoxy-1-propylamin.

In einer besonderen Ausführungsform wird als Lösungsmittel eine Kombination aus mindestens einem Alkoxyalkylamin mit mindestens einem C₁-C₄-Alkohol und/oder C₁-C₃-Diol und/oder soweit Mischbarkeit gegeben ist, mit Wasser eingesetzt. Der C₁-C₄-Alkohol kann Methanol, Ethanol, 1-Propanol, 2-Propanol, 1-Butanol, 2-Butanol oder tert.-Butanol sein; das C₁-C₃-Diol umfaßt 1,2-Ethandiol, 1,2-Propandiol und 1,3-Propandiol. Das Verhältnis von Alkoxyalkylamin zum C₁-C₄-Alkohol und/oder C₁-C₃-Diol und/oder Wasser ist keiner Beschränkung unterworfen und wird im allgemeinen von der Löslichkeit der Base im Reaktionsgemisch und der Reaktivität des eingesetzten 2-Nitrotoluols bestimmt. Normalerweise wird der Alkoxyalkylaminanteil im Lösungsmittelgemisch 30 bis 95 Gew.-%, bevorzugt 50 bis 95 Gew.-% betragen.

In besonders bevorzugter Weise wird das erfindungsgemäße Verfahren in Gemischen aus Alkoxyalkylaminen der Formel

R₃O-(CH₂)ₙ-NH₂ (IV)

worin
- R₃: für Methyl oder Ethyl und
- n: für 1-3 stehen,
und Methanol als Lösungsmittel durchgeführt.

Als starke Basen kommen Verbindungen der Alkali- und Erdalkalimetalle in Frage, beispielsweise Metallhydroxide, Metallalkoholate und Metallamide und, soweit es sich um Alkoholate und Amide handelt, von Aluminium. Als wichtige Alkali- und Erdalkalimetalle seien beispielsweise Natrium, Kalium, Lithium, Calcium, Magnesium genannt. Als Alkoholate seien beispielsweise das Methylat, Ethylat, Isopropylat, 2-Butanolat, tert.-Butanolat und das Propylenglykolat aufgeführt. Unter den genannten Basen sind die Hydroxide und Alkoholate bevorzugt; insbesondere bevorzugt sind Natriumhydroxid und Kaliumhydroxid und Natriummethylat. Die genannten Basen können einzeln oder in beliebigem Gemisch untereinander eingesetzt werden.

Die starke Base hat lediglich katalytische Funktion. Entsprechend kann die Oxidation von 2-Nitrotoluolen zu 2-Nitrobenzaldehyden nach dem erfindungsgemäßen Verfahren bereits durch den Zusatz katalytischer Mengen der starken Basen in Gang gesetzt werden. Trotzdem kann es in bestimmten Fällen von Vorteil sein stöchiometrische oder gar überstöchiometrische Mengen der starken Basen einzusetzen. Welche Mengen der starken Basen letztlich notwendig sind hängt von der jeweiligen Reaktivität des eingesetzten 2-Nitrotoluols der Formel (II) ab.

Im allgemeinen kann die Aufwandmenge der starken Basen zwischen 0,1 und 10 Basenäquivalenten pro mol 2-Nitrotoluol, bevorzugt zwischen 0,2 und 5 Basenäquivalenten, besonders bevorzugt zwischen 0,2 und 3 Basenäquivalenten pro mol 2-Nitrotoluol variieren.

Die selektive Oxidation von 2-Nitrotoluolen zu 2-Nitrobenzaldehyden durch Behandeln von 2-Nitrotoluolen mit Sauerstoff in Gegenwart von starken Basen ist eine sehr unerwartete Reaktion, da bei der Oxidation von Toluolen wie z.B. Toluol oder Xylol mit Sauerstoff normalerweise der gebildete Aldehyd unter den Reaktionsbedingungen rasch zur entsprechenden Benzoesäure weiterreagiert und die Aldehydselektivität entsprechend nur sehr gering ist. Die Reaktion ist darüberhinaus umso überraschender als nach EP-A 207 123 beim Behandeln von 2-Nitrotoluol und von 4-Nitrotoluol mit Sauerstoff in Gegenwart von starken Basen als Hauptprodukte 2,2'-Dinitrobibenzyl bzw. 4,4'-Dinitrobibenzyl entstehen.

Die Ausführung des erfindungsgemäßen Verfahrens unter Zusatz eines Katalysators kann vorteilhaft sein, ist aber nicht zwingend notwendig. Als Katalysatoren kommen insbesondere Verbindungen der Übergangsmetalle, beispielsweise von Co, Mn, Cr, Fe, Ni, Cu, V, Ru zum Einsatz. Mögliche Einsatzformen dieser Metalle sind deren Salze anorganischer Säuren, beispielsweise die Metallfluoride, -chloride, -sulfate, -nitrate, -carbonate, - phosphate; die Metalloxide und Metallhydroxide; die Metallsalze organischer Säuren, beispielsweise die Metallacetate, -oxalate, -phenolate, -benzoate, - salicylate; Komplexe dieser Metalle beispielsweise mit Acetylaceton, N,N'-Disalicyliden-ethylendiamin, Tetraphenylporphin und Phthalocyanin. Von besonderem Interesse sind unter den Metallkatalysatoren die Verbindungen des Mangans.

Die Menge an Metallkatalysator kann über einen weiten Bereich variieren. Normalerweise sind Aufwandmengen von 0,0001 bis 0,05, bevorzugt 0,0005 bis 0,02 Moläquivalente pro mol 2-Nitrotoluol ausreichend. Selbstverständlich kann der katalytische Effekt der Metallkatalysatoren auch beim Einsatz höherer Mengen an Metallkatalysator vorteilhaft genutzt werden.

Der als Oxidationsmittel für das erfindungsgemäße Verfahren verwendete Sauerstoff kann in reiner Form oder in verdünnter Form, beispielsweise in Form von Sauerstoff enthaltenden Gasen, eingesetzt werden. Die wirtschaftlich günstigste Form des erfindungsgemäß einzusetzenden Sauerstoffs ist die atmosphärische Luft. Der Druck des Sauerstoffs bzw. des Sauerstoff enthaltenden Gases ist keiner besonderen Einschränkung unterworfen und kann zwischen 0,5 und 20 bar liegen, vorzugsweise zwischen 0,8 und 10 bar, besonders bevorzugt bei 0,8 bis 3 bar. Der Sauerstoffgehalt ist bei Verwendung von Sauerstoff enthaltenden Gasen ebenfalls keiner Beschränkung unterworfen.

Gehalt und Druck des Sauerstoff enthaltenden Gases richtet sich vor allem nach der Umsetzungsgeschwindigkeit und der erzielbaren Selektivität. Die für den Einzelfall günstigsten Begasungsbedingungen lassen sich durch einfache Vorversuche ermitteln.

Es ist von Vorteil, den Sauerstoff oder die Luft im Reaktionsgemisch fein zu verteilen, beispielsweise unter Verwendung von Düsen oder Fritten. Der Sauerstoff oder die Luft kann jedoch auch unter Verwendung geeigneter Rührer durch kräftiges Rühren in das Reaktionsgemisch eingezogen werden.

Die Reaktionstemperatur für das erfindungsgemäße Verfahren kann zwischen - 50°C und +50°C variieren. Bevorzugt wird jedoch bei einer Temperatur zwischen -30 und +30°C gearbeitet.

Die Isolierung der 2-Nitrobenzaldehyde aus den beim erfindungsgemäßen Verfahren anfallenden Reaktionsgemischen erfolgt nach den bekannten verfahrenstechnischen Grundoperationen wie z.B. Destillation oder Extraktion und richtet sich in erster Linie nach den bei der Oxidation verwendeten Lösungsmitteln. Wurden bei der Oxidation als Lösungsmittel Verbindungen mit primären Aminogruppen eingesetzt, so liegen die 2-Nitrobenzaldehyde primär als Aldehydimine vor. Aus solchen Reaktionsgemischen können die Lösungsmittel beispielsweise durch Destillation abgetrennt werden. Gegegebenenfalls kann es zweckmäßig sein vor Abtrennung der Lösungsmittel überschüssige starke Base mit Säuren zu neutralisieren. Das Rohgemisch nach Abtrennung der Lösungsmittel wird mit Wasser und katalytischen Mengen Mineralsäure versetzt, um evtl. gebildete Imine zu hydrolysieren, und der 2-Nitrobenzaldehyd beispielsweise durch Destillation, Wasserdampfdestillation oder Extraktion isoliert. Vorteilhaft kann die Isolierung und Reinigung des 2-Nitrobenzaldehyds auch über das Bisulfit-Addukt durchgeführt werden.

Die Prozentangaben der nachfolgenden Beispiele beziehen sich jeweils auf das Gewicht.

### Beispiel 1

In einem 250 ml Glasreaktor wurden bei -5°C 13,7 g 2-Nitrotoluol, 0,10 g MnSO₄·H₂O und 100 g 2-Methoxyethylamin vorgelegt. Anschließend wurde unter Begasung der Lösung mit reinem Sauerstoff unter Normaldruck eine Lösung von 6,6 g KOH in 12 g Methanol während 1 h zudosiert. Nach beendeter Alkalidosierung ließ man noch 3,5 h nachoxidieren bis zu einer O₂-Gesamtaufnahme von 2,0 l und neutralisierte dann das überschüssige Alkali durch Zugabe von 80%iger Schwefelsäure. Die Temperatur im Reaktionsgemisch wurde während der gesamten Reaktion bei -5°C gehalten. Die Produktisolierung begann mit dem Abdestillieren der Lösungsmittel 2-Methoxyethylamin und Methanol. Der Rückstand aus der Lösungsmitteldestillation wurde mit ca. 150 ml Wasser versetzt, mit 80%iger Schwefelsäure auf pH 1 angesäuert und die Wasserphase dreimal mit Methylenchlorid extrahiert. Die Methylenchloridphase wurde über Natriumsulfat getrocknet und das Lösungsmittel abdestilliert. Als Rückstand blieben 14,3 g dunkel gefärbtes Öl, das nach GC-Analyse einen Gehalt von 20,8 % 2-Nitrotoluol und 50,2 % 2-Nitrobenzaldehyd aufwies.

| | |
|---|---|
| Umsatz 2-Nitrotoluol | 78,3 % |
| Selektivität 2-Nitrobenzaldehyd | 60,8 % |

### Beispiel 2

Durchführung wie Beispiel 1 mit dem Unterschied, daß bei -10°C oxidiert wurde und daß als starke Base 18,0 g einer 30%igen Lösung von Natriummethanolat in Methanol zudosiert wurden. Reaktionsabbruch nach Aufnahme von 2,5 l O₂; Reaktionsdauer 5,5 h.
Aufarbeitung wie in Beispiel 1:

| | |
|---|---|
| Rohprodukt | 15,2 g dunkel gefärbtes Öl |
| Umsatz 2-Nitrotoluol | 83 % |
| Selektivität 2-Nitrobenzaldehyd | 55,9 % |

### Beispiel 3

Durchführung wie Beispiel 1 mit dem Unterschied, daß bei 0°C ohne MnSO₄-Zusatz gearbeitet wurde und daß als starke Base 35,0 g einer 30 %igen Lösung von Natriummethanolat in Methanol zudosiert wurden. Reaktionsabbruch nach 4 h bei 2,5 l O₂-Aufnahme. Aufarbeitung wie in Beispiel 1.

| | |
|---|---|
| Rohprodukt | 15,2 g dunkel gefärbtes Öl |
| Umsatz 2-Nitrotoluol | 92,1 % |
| Selektivität 2-Nitrobenzaldehyd | 40,4 % |

### Beispiel 4

Durchführung wie Beispiel 1 mit dem Unterschied, daß 2-Methoxyethylamin durch 100 g 3-Methoxypropylamin ersetzt wurde. Reaktionsabbruch nach 6 h bei 0,8 l O₂-Aufnahme.

Aufarbeitung wie in Beispiel 1

| | |
|---|---|
| Rohprodukt | 14,1 g dunkel gefärbtes Öl |
| Umsatz 2-Nitrotoluol | 30,0 % |
| Selektivität 2-Nitrobenzaldehyd | 62,3 % |

### Beispiel 5

Durchführung wie Beispiel 1 mit dem Unterschied, daß 2-Methoxyethylamin durch 100 g 3-Ethoxypropylamin ersetzt wurde und daß bei +5°C oxidiert wurde. Reaktionsabbruch nach 5,5 h bei 0,9 l O₂-Aufnahme

Aufarbeitung wie in Beispiel 1

| | |
|---|---|
| Rohprodukt | 14,4 g dunkel gefärbtes Öl |
| Umsatz 2-Nitrotoluol | 33,6 % |
| Selektivität 2-Nitrobenzaldehyd | 59,2 % |

### Beispiel 6

In einem Glasreaktor wurden bei -15°C 13,7 g 2-Nitrotoluol, 0,10 g MnSO₄ x H₂O und 100 g 2-Methoxyethylamin vorgelegt. Anschließend wurden unter Begasung der Lösung mit reinem Sauerstoff unter Normaldruck 4,0 g Natriumhydroxidpulver zugesetzt. Nach einer wenige Minuten dauernden Induktionsperiode setzte die O₂-Aufnahme ein. Die Temperatur im Reaktionsgemisch wurde während der gesamten Reaktion bei -15°C gehalten. Abbruch der Reaktion nach 8 h bei einer O₂-Gesamtaufnahme von 2,3 l durch Neutralisation des überschüssigen Alkali mit 80 %iger H₂SO₄.

Aufarbeitung wie in Beispiel 1

| | |
|---|---|
| Rohprodukt | 15,4 g dunkel gefärbtes Öl |
| Umsatz 2-Nitrotoluol | 83,5 % |
| Selektivität 2-Nitrobenzaldehyd | 56,8 % |

## Patentansprüche

1. Verfahren zur Herstellung von 2-Nitrobenzaldehyden der allgemeinen Formel 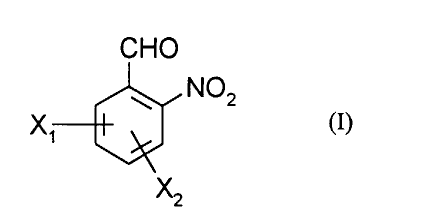 worin
X₁ und X₂ entweder unabhängig voneinander für Wasserstoff oder Halogen stehen oder einer der Substituenten für Nitro steht und der andere Substituent dann für Wasserstoff steht,
durch Oxidation von 2-Nitrotoluolen der allgemeinen Formel 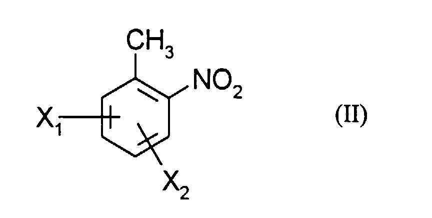 worin
X₁ und X₂ die oben angegebene Bedeutung haben
dadurch gekennzeichnet, daß die Oxidation mit Sauerstoff oder einem Sauerstoff enthaltenden Gas in Gegenwart mindestens eines Alkoxyalkylamins als Lösungsmittel und in Gegenwart von starken Basen durchgeführt wird.

2. Verfahren gemäß Anspruch 1, dadurch gekennzeichnet, daß als Lösungsmittel Alkoxyalkylamine der Formel (III) 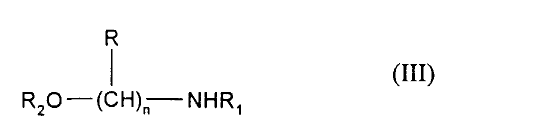 worin
R und R₁ unabhängig voneinander für Wasserstoff oder C₁-C₄-Alkyl stehen,
R₂ für C₁-C₄-Alkyl steht und
n für 1 bis 6 steht,
eingesetzt werden.

3. Verfahren gemäß Anspruch 1, dadurch gekennzeichnet, daß als Lösungsmittel eine Kombination aus mindestens einem Alkoxyalkylamin und mindestens einem C₁-C₄-Alkohol und/oder C₂-C₃-Diol und/oder Wasser eingesetzt wird.

4. Verfahren gemäß Anspruch 1, dadurch gekennzeichnet, daß ein Gemisch aus Alkoxyalkylaminen der Formel IV
R₃O-(CH₂)ₙ-NH₂ (IV)
worin
R₃ für Methyl oder Ethyl und
n für 1-3 stehen,
und Methanol als Lösungsmittel eingesetzt wird.

5. Verfahren gemäß Anspruch 1, dadurch gekennzeichnet, daß als starke Basen einer oder mehrere Vertreter aus der Gruppe der Hydroxide, Alkoholate und Amide von Alkali- und Erdalkalimetallen und Alkoholate und Amide von Aluminium in einer Menge von 0,1 bis 10 Basenäquivalenten, bevorzugt 0,2 bis 5 Basenäquivalenten, besonders bevorzugt zwischen 0,2 bis 3 Basenäquivalenten pro mol 2-Nitrotoluol eingesetzt werden.

6. Verfahren gemäß Anspruch 1, dadurch gekennzeichnet, daß als starke Basen Natriumhydroxid, Kaliumhydroxid oder Natriummethanolat eingesetzt werden.

7. Verfahren gemäß Anspruch 1, dadurch gekennzeichnet, daß es in Gegenwart einer oder mehrerer Katalysatoren aus der Gruppe der Übergangsmetallverbindungen, insbesondere des Co, Mn, Cr, Fe, Ni, Cu, V, Ru durchgeführt wird.

8. Verfahren gemäß Anspruch 1, dadurch gekennzeichnet, daß es in Gegenwart einer Verbindung des Mangans als Katalysator in einer Menge von 0,0001 bis 0,05 Moläquivalenten, bevorzugt 0,005 bis 0,02 Moläquivalenten, bezogen auf das 2-Nitrotoluol, durchgeführt wird.

9. Verfahren gemäß Anspruch 1, dadurch gekennzeichnet, daß X₁ und X₂ in Formeln (I) und (II) für Wasserstoff stehen.

10. Verfahren gemäß Anspruch 1, dadurch gekennzeichnet, daß es bei einer Temperatur zwischen -50°C und +50°C, bevorzugt zwischen -30°C und +30°C durchgeführt wird.

## Claims

1. Process for the preparation of 2-nitrobenzaldehydes of the general formula 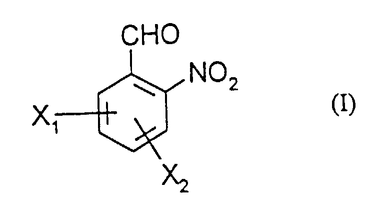 in which
X₁ and X₂ either independently of one another represent hydrogen or halogen or one of the substituents represents nitro and the other substituent then represents hydrogen,
by oxidation of 2-nitrotoluenes of the general formula 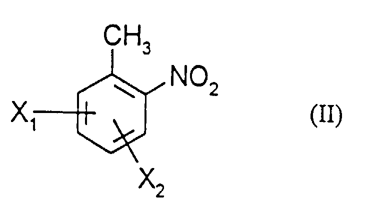 in which
X₁ and X₂ have the meaning indicated above
characterized in that the oxidation with oxygen or an oxygen-containing gas is carried out in the presence of at least one alkoxyalkylamine as a solvent and in the presence of strong bases.

2. Process according to Claim 1, characterized in that the solvents employed are alkoxyalkylamines of the formula (III) 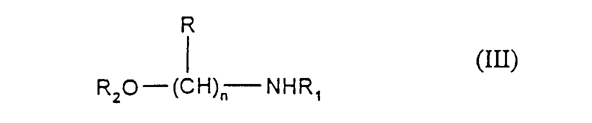 in which
R and R₁ independently of one another represent hydrogen or C₁-C₄-alkyl,
R₂ represents C₁-C₄-alkyl and
n represents 1 to 6.

3. Process according to Claim 1, characterized in that the solvent employed is a combination of at least one alkoxyalkylamine and at least one C₁-C₄-alcohol and/or C₂-C₃-diol and/or water.

4. Process according to Claim 1, characterized in that a mixture of alkoxyalkylamines of the formula IV
R₃O-(CH₂)ₙ-NH₂ (IV)
in which
R₃ represents methyl or ethyl and
n represents 1-3,
and methanol as the solvent is employed.

5. Process according to Claim 1, characterized in that the strong bases employed are one or more representatives from the group consisting of the hydroxides, alkoxides and amides of alkali metals and alkaline earth metals and alkoxides and amides of aluminium in an amount from 0.1 to 10 base equivalents, preferably 0.2 to 5 base equivalents, particularly preferably 0.2 to 3 base equivalents, per mole of 2-nitrotoluene.

6. Process according to Claim 1, characterized in that the strong bases employed are sodium hydroxide, potassium hydroxide or sodium methoxide.

7. Process according to Claim 1, characterized in that it is carried out in the presence of one or more catalysts from the group consisting of the transition metal compounds, in particular of Co, Mn, Cr, Fe, Ni, Cu, V and Ru.

8. Process according to Claim 1, characterized in that it is carried out in the presence of a compound of manganese as a catalyst in an amount from 0.0001 to 0.05 mol equivalents, preferably 0.005 to 0.02 mol equivalents, relative to the 2-nitrotoluene.

9. Process according to Claim 1, characterized in that X₁ and X₂ in formulae (I) and (II) represent hydrogen.

10. Process according to Claim 1, characterized in that it is carried out at a temperature between -50°C and +50°C, preferably between -30°C and +30°C.

## Revendications

1. Procédé de préparation de 2-nitrobenzaldéhydes de formule générale 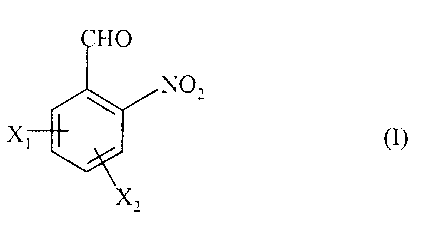 où
X₁ et X₂ représentent soit indépendamment l'un de l'autre l'hydrogène ou un halogène soit l'un des substituants représente le nitro et l'autre substituant est alors l'hydrogène,
par oxydation de 2-nitrotoluènes de formule générale 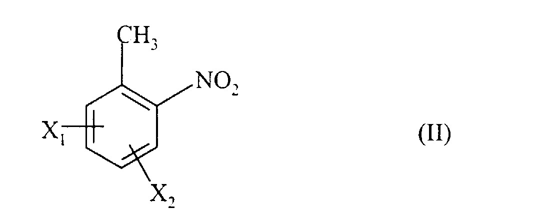 où
X₁ et X₂ ont la signification donnée ci-dessus
caractérisé en ce qu'on réalise l'oxydation avec l'oxygène ou avec un gaz contenant de l'oxygène en présence d'au moins une alcoxyalkylamine comme solvant et en présence de bases fortes.

2. Procédé selon la revendication 1, caractérisé en ce qu'on utilise comme solvants des alcoxyalkylamines de formule (III) 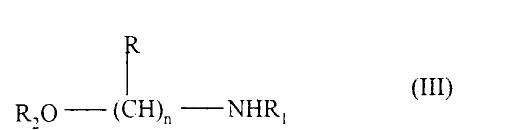 où
R et R₁ représentent indépendamment l'un de l'autre l'hydrogène ou un alkyle C₁-C₄,
R₂ représente un alkyle C₁-C₄ et
n représente 1 à 6.

3. Procédé selon la revendication 1, caractérisé en ce qu'on utilise comme solvant une combinaison d'au moins une alcoxyalkylamine et au moins un alcool C₁-C₄ et/ou un diol C₂-C₃ et/ou de l'eau.

4. Procédé selon la revendication 1, caractérisé en ce qu'on utilise un mélange d'alcoxyalkylamines de formule IV
R₃O-(CH₂)ₙ-NH₂ (IV)
où
R₃ représente un méthyle ou un éthyle et
n représente 1-3, et
le méthanol comme solvants.

5. Procédé selon la revendication 1, caractérisé en ce qu'on utilise comme bases fortes un ou plusieurs représentants du groupe des hydroxydes, des alcoolates et des amides de métaux alcalins et alcalino-terreux et des alcoolates et des amides d'aluminium en une quantité de 0,1 à 10 équivalents de base, de préférence 0,2 à 5 équivalents de base, surtout entre 0.2 et 3 équivalents de base par mol de 2-nitrotoluène.

6. Procédé selon la revendication 1, caractérisé en ce qu'on utilise comme bases fortes de l'hydroxyde de sodium, de l'hydroxyde de potassium ou du méthanolate de sodium.

7. Procédé selon la revendication 1, caractérisé en ce qu'on réalise ce procédé en présence d'un ou plusieurs catalyseurs du groupe des composés des métaux de transition, notamment des Co, Mn, Cr, Fe, Ni, Cu, V, Ru.

8. Procédé selon la revendication 1, caractérisé en qu'on le réalise en présence d'un composé du manganèse comme catalyseur en une quantité de 0,0001 à 0,05 équivalents molaires, de préférence 0,005 à 0,02 équivalents molaires, par rapport au 2-nitrotoluène.

9. Procédé selon la revendication 1, caractérisé en ce que X₁ et X₂ dans les formules (I) et (II) représentent l'hydrogène.

10. Procédé selon la revendication 1, caractérisé en ce qu'on le réalise à une température comprise entre -50°C et +50°C, de préférence entre -30°C et +30°C.
